# EUROPEAN PATENT APPLICATION

(11) **EP 4 375 289 A1**
(43) Date of publication of application: **29.05.2024**
(21) Application number: 22208808.0
(22) Date of filing: 22.11.2022
(51) Int. Cl.: C07K 7/08, A61P 31/04, A61K 38/10

(54) **PEPTIDE WITH ANTIMICROBIAL ACTIVITY**

(71) Applicant: Freie Universität Berlin, 14195 Berlin (DE)
(72) Inventor: KOKSCH, Beate, 14532 Kleinmachnow (DE); CHOWDHARY, Suvrat, 14059 Berlin (DE); FULDE, Marcus, 14109 Berlin (DE); PELZER, Tim, 10553 Berlin (DE)
(74) Representative: Maikowski & Ninnemann Patentanwälte Partnerschaft mbB

(57) **Abstract**

The invention refers to a peptide with antimicrobial activity comprising the following sequence

Y¹-[R-X¹-R-X²-R-dL-dF-[2]Abz-dA-R-X²-R-X¹-R]-Y²

Wherein R is D-or L- Arginine or an Arginine analogue, dL is D-Leucine, dF is D-Phenylalanine, [2]Abz is 2-Aminobenzoic acid, dA is D-Alanine, X¹ is FₐH_{b}C - (F_{c}H_{d}C)ₙ -CH₂-CHNH₂CO₂H, wherein a is 0,1, 2, 3; b is 0, 1, 2, 3; wherein the sum of a and b is always 3; c is 0, 1, 2; d is 0, 1, 2; wherein the sum of c and d is always 2; Wherein n is 0-5, X² is L-Tryptophan (W), D-Tryptophan or a Tryptophan analogue or X¹, Y¹ is Acetyl or H or any suitable protective group; and Y² is NH2 or OH or any suitable protective group.

## Description

The present invention relates to a peptide with antimicrobial activity.

### Description

Peptides can adopt a multitude of biologically active conformations encouraging biomolecular recognition. The β-turn motif is one of the most common structural features in peptides and proteins and allows a stabilization of its pattern by a nearly 180°C reverse turn. This folding pattern was found for a wide range of naturally occurring antimicrobial peptides (AMPs) (G. Wang, X. Li and Z. Wang, Nucleic Acids Res, 2016, 44, D1087-1093; Y. Huan, Q. Kong, H. Mou and H. Yi, Frontiers in Microbiology, 2020, 11; J.-P. S. Powers and R. E. W. Hancock, Peptides, 2003, 24, 1681-1691). Thus, the design of *de novo* designed AMPs is of paramount interest as the growing resistance of pathogenic microbes towards currently used antibiotics is a rapidly growing threat for the global health care management.

The incorporation of fluorine into peptide-based antibiotics is considered as promising approach for providing AMPs. While rarely found in nature, this element is mainly used as a tool for fine-tuning the biophysical properties of peptides and proteins (A. A. Berger, J.-S. Völler, N. Budisa and B. Koksch, Accounts of Chemical Research, 2017, 50, 2093-2103; M. Salwiczek, E. K. Nyakatura, U. I. M. Gerling, S. Ye and B. Koksch, Chemical Society Reviews, 2012, 41, 2135-2171).

Substitution of a single hydrogen atom with fluorine within an amino acid residue leads to minimum steric requirements. Simultaneously, the hydrophobic nature of peptides is considered to be directly controlled by the degree of fluorination and so its bioactivity (J. N. Sloand, M. A. Miller and S. H. Medina, Peptide Science, 2021, 113, e24184).

However, the physicochemical benefits of fluorous amino acids in peptide-based antibiotics are scarcely reported. Early reports in 2006 emphasized trifluoromethylated amino acids to enhance the antibacterial activity of cationic oligopeptides towards Gram-negative and Gram-positive bacterial strains (D. Giménez, C. Andreu, M. I. d. Olmo, T. Varea, D. Diaz and G. Asensio, Bioorganic & Medicinal Chemistry, 2006, 14, 6971-6978).

In particular, the perfluorinated amino acid L-5,5,5,5',5',5'-hexafluoroleucine (hFLeu), has been extensively studied in AMP scaffolds. When incorporated into *pexiganan*, a 22-amino acid peptide-based drug, overall bioactivity was retained by neglecting peptide-induced hemolysis up to a concentration of 250 µg/mL (L. M. Gottler, H. Y. Lee, C. E. Shelburne, A. Ramamoorthy and E. N. Marsh, Chembiochem, 2008, 9, 370-373).

In contrast, hfLeu-containing variants of the β-hairpin peptide *protegrin-1* led to an impaired antimicrobial activity against common pathogenic bacterial strains compared to its native sequence, as well as increased rates of hemolysis (L. M. Gottler, R. de la Salud Bea, C. E. Shelburne, A. Ramamoorthy and E. N. G. Marsh, Biochemistry, 2008, 47, 9243-9250).

Meng *et al.* introduced a library of hFLeu-containing *buforin-2* and *magainin-2* mutants, leading to a comprehensive improvement of antimicrobial activity but also partially increased peptide-induced hemolysis (H. Meng and K. Kumar, Journal of the American Chemical Society, 2007, 129, 15615-15622).

However, recent studies focusing on fluorous phenylalanine derivatives in AMPs reported an overall loss in antibiotic strength in comparison to the non-fluorinated variant (S. C. Setty, S. Horam, M. Pasupuleti and W. Haq, International Journal of Peptide Research and Therapeutics, 2017, 23, 213-225).

Hence, the role of fluorination in antimicrobial peptides is controversially discussed. In a similar manner, a lack of reports about the impact of fluorinated amino acids on the proteolytic resistance of such peptide scaffolds obscures their utilization as engineering tools for the development of advanced antibiotic compounds.

It is an object of the present invention to provide novel peptides with antimicrobial activity and proteolytic stability that eventually are applicable in the treatment of microbial infections.

This object is solved with a peptide having the features of claim 1.

Accordingly, a peptide is provided comprising the following sequence
Y¹-[R-X¹-R-X²-R-dL-dF-[2]Abz-dA-R-X²-R-X¹-R]-Y² Wherein
- R is D-or L- Arginine or an Arginine analogue, dL is D-Leucine, dF is D-Phenylalanine, [2]Abz is 2-Aminobenzoic acid, dA is D-Alanine,
- X¹ is FₐH_{b}C - (F_{c}H_{d}C)ₙ -CH₂-CHNH₂CO₂H, wherein
   a is 0,1,2,3; b is 0, 1, 2, 3; wherein the sum of a and b is always 3;
   c is 0, 1, 2; d is 0, 1, 2; wherein the sum of c and d is always 2;
   Wherein n is 0-5,
- X² is L-Tryptophan (W), D-Tryptophan or a Tryptophan analogue, or X¹,
- Y¹ is Acetyl,-H or any suitable protective group; and
- Y² is -NH₂,-OH or any suitable protective group.

The peptide according to the invention has been shown to combine both, antimicrobial activity and proteolytic stability. The incorporation of a wide range of unnatural aliphatic building blocks with distinctive degrees of fluorination retained secondary structure formation and functioned as an approach to fine-tune the hydrophobic properties of each peptide. Minimal inhibitory concentration (MIC) screening revealed a correlation between fluorine-enhanced hydrophobicity and superior activities. All peptides were found to be barely hemolytic, low toxic, and susceptible for enzymatic degradation.

In an embodiment of the present peptide a is 2 or 3; b is 0 or 1; wherein the sum of a and b is always 3; c is 1 or 2; d is 0 or 1, wherein the sum of c and d is always 2, n is 0, 1, 2, preferably n is 0 or 1; and X² is L-Tryptophan (W), D-Tryptophan, a Tryptophan analogue, preferably L-Tryptophan (W), or X¹.

In a further embodiment of the present peptide, a is 3; b is 0; c is 2; d is 0, n is 0 or 1; and X² is L-Tryptophan (W), D-Tryptophan or a Tryptophan analogue, preferably L-Tryptophan (W), or X¹.

In yet a further embodiment of the present peptide a is 3; b is 0; c is 2; d is 0, n is 0 or 1; X² is L-Tryptophan (W), D-Tryptophan or a Tryptophan analogue, preferably L-Tryptophan (W), Y¹ is H; and Y² is OH.

In an embodiment, the Tryptophan analogue used in the present peptide may be one of the following β-Homo Trp-OH or 5-fluoro-L-Trp-OH.

In an embodiment, R may be an Arginine analog such as β-HomoArg(Pbf)-OH or D-Arg(Pbf)-OH.

In still another embodiment, X¹ may be 2-Aminobutyric acid (Abu) or an analogue thereof or a valine analogue, such as β-HomoVaI-OH, (R)-4-amino-5-methylhexanoic acid or D-Val-OH.

In still a further preferred variant of the present peptide R is L-Arginine.

As mentioned above, Y¹ and Y² may be also any suitable protective group, in particular any suitable group protective against proteolysis. Such a protective group may be one of the following:
- Benzoyl (Bz), Benzyl (BnBn, BnlBnl), ββ-Methoxyethoxymethyl ether (MEM), Dimethoxytrityl, [bis-(4-methoxyphenyl)phenylmethyl] (DMT), Methoxymethyl ether (MOM), Methoxytrityl [(4-methoxyphenyl)diphenylmethyl, MMT), p-Methoxybenzyl ether (PMB), Pivaloyl (Piv)(Piv), Tetrahydropyranyl (THP), Trityl (triphenylmethyl, TrTr),
- Carbobenzyloxy (Cbz) group, p-Methoxybenzyl carbonyl (Moz or MeOZ) group, tert-Butyloxycarbonyl (BOC) group, 9-Fluorenylmethyloxycarbonyl (FMOC) group, Carbamate group, p-Methoxybenzyl (PMB), 3,4-Dimethoxybenzyl (DMPM), p-methoxyphenyl (PMP) group, Tosyl (Ts) group, , L-Pyroglutamic Acid.

In a preferred embodiment Y¹ is Acetyl or H, Y² is -NH₂, -OH or L-Pyroglutamic Acid, wherein Acteyl, -NH₂ and , L-Pyroglutamic Acid are mostly preferred.

As illustrated above, the present peptide comprises a non-natural amino acid, in particular a fluorine containing non-natural amino acids, in particular a non-natural glycine derivative. The length of the side chain in the glycine derivative may vary and can include any of methyl, ethyl, propyl, butyl or pentyl side chains. As stated, the side chain preferably contains fluorine, but may also be free of fluorine. In the latter case the non-natural amino acid is 2-Aminobutyric acid (Abu).

The non-natural amino acids may be selected from one of the following:

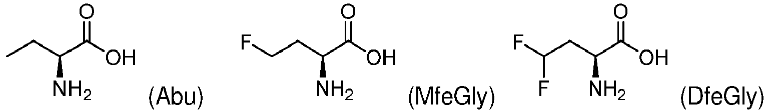

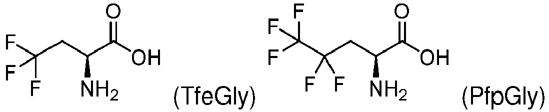

Particular embodiments of the invention are one of the following peptides:

| **Peptide** | **Sequence** |
|---|---|
| **AbuR14** | Ac-R-Abu-R-Abu-R-[I-f-[2]Abz-a]-R-Abu-R-Abu-R-NH2 |
| **MfeGlyR14** | Ac-R-MfeGly-R-MfeGly-R-[I-f-[2]Abz-a]-R-MfeGly-R-MfeGly-R-NH2 |
| **DfeGlyR14** | Ac-R-DfeGly-R-DfeGly-R-[I-f-[2]Abz-a]-R-DfeGly-R-DfeGly-R-NH2 |
| **TfeGlyR14** | Ac-R-TfeGly-R-TfeGly-R-[I-f-[2]Abz-a]-R-TfeGly-R-TfeGly-R-NH2 |
| **SAJO-2** | H-R- Val-R- Trp-R-[I-f-[2]Abz-a]-R-Trp-R- Val-R-OH |
| **SAJO-Abu** | H-R-Abu-R-Trp-R-[I-f-[2]Abz-a]-R-Trp-R-Abu-R-OH |
| **SAJO-TfeGly** | H-R-TfeGly-R-Trp-R-[I-f-[2]Abz-a]-R-Trp-R-TfeGly-R-OH |
| **SAJO-PfpGly** | H-R-PfpGly-R-Trp-R-[I-f-[2]Abz-a]-R-Trp-R-PfpGly-R-OH |

The peptides of the present invention may be used as an antimicrobial substance, in particular as an antibiotic or antifungal.

In particular, the peptides of the present invention may be used in a method of treatment of bacterial infections by gram-negative or gram-positive bacterial strains.

The bacterial infection may be an infection caused by one of the genus Acinetobacter, Bordatella, Borellia, Brucella, Camphylobacter, Chlamydia, Chlamydophila, Enterobacter, Escherichia, Francisella, Haemophilus, Helicobacter, Klebsiella, Legionella, Leptospira, Morganella Moraxella, Neisseria, Proteus, Pseudomonas, Rickettsia, Shigella, Salmonella, Stenotrophomonas, Treponema or Yersinia,

In a further embodiment the bacterial infection is an infection caused
- by a gram-positive bacterium, particularly an infection by one of the genus Bacillus, Chlostridium, Corynebacterium, Enterococcus, Listeria, Micrococcus, Staphylococcus or Streptococcus, further in particular by one of the genus of Staphylococcus, Streptococcus, Bacillus or Micrococcus or
- by a bacterium of the family of Mycobacteriaceae, in particular of the genus Mycobacterium, further in particular an infection by one of *Mycobacterium tuberculosis, Mycobacterium leprae, Mycobacterium ulcerans* or *Mycobacterium avium,* or
- by a bacterium of the family of Mycoplasmataceae, in particular of the genus Mycoplasma, further in particular an infection by *Mycoplasma pneumonia.*

In a preferred embodiment, the bacterial infection may be an infection caused by one of the genus of Staphylococcus, Escherichia, Pseudomonas, Klebisella, in particular caused by one of *Staphylococcus aureus, Escherichia coli, Escherichia faecalis, Klebisella pneumonia, Pseudomonas aeruginosa.*

In a preferred embodiment the present peptide, in particular peptide Sajo-PfpGly, shows antibacterial activity against antibiotic resistant bacterial strains, in particular antibiotic resistant *E.coli* strains.

It is also possible that the peptide is used for treating fungal infection, for example an infection caused by *Candida albicans.*

The peptide according to the invention is obtained by solid phase synthesis. Accordingly, the method for manufacturing a peptide according to the invention comprises the following steps:
a) synthesizing a peptide by solid phase peptide synthesis on a solid phase peptide synthesis resin, the peptide comprising the structure as described previously,
b) cleaving the synthesized peptide from the solid phase peptide synthesis resin;
c) optionally purifying the synthesized and cleaved peptide.

The synthesis method is described in more detail below.

The present invention is explained now in more detail by means of the following examples.

### Synthesis of fluorinated amino acids MfeGlv & DfeGly

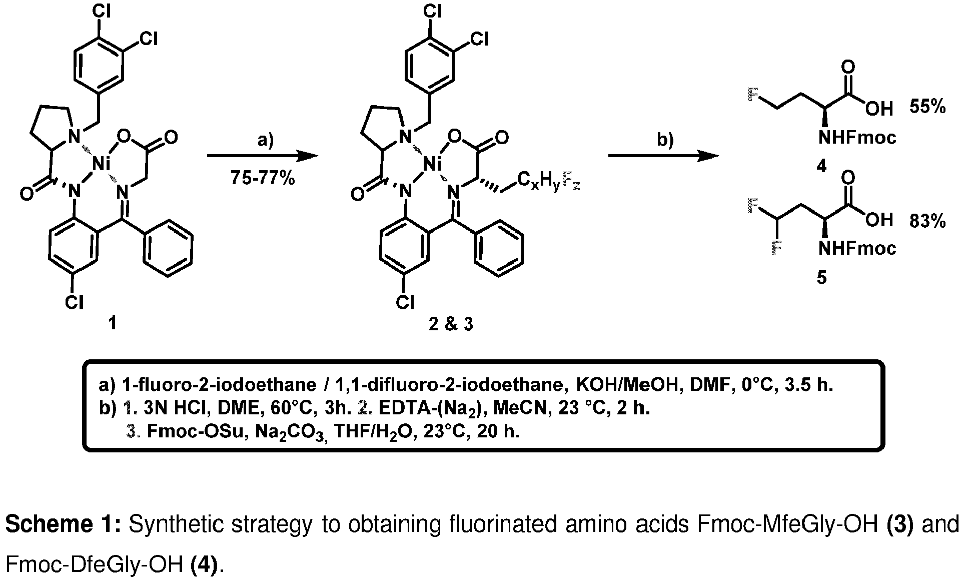

The fluorinated amino acids Fmoc-MfeGly-OH (5) and Fmoc-DfeGly-OH (6) were obtained through a five-step synthetic strategy based on the stereoselective alkylation of a chiral Ni-ligand 1 introduced by Soloshonok *et al.* A standard protocol for the synthesis of 1 can be found in literature. The alkylation of 1 and subsequent release and Fmoc-protection of amino acids are based on an optimized strategy developed by Hohmann *et al.* for the gram-scale synthesis of a wide range of aliphatic fluorinated derivatives. The gram-scale synthesis of Fmoc-TfeGly-OH was proceeded according to Chowdhary *et al.* (S. Chowdhary, R. F. Schmidt, A. K. Sahoo, T. tom Dieck, T. Hohmann, B. Schade, K. Brademann-Jock, A. F. Thünemann, R. R. Netz, M. Gradzielski, B. Koksch, Nanoscale 2022, 14, 10176-10189). The amino acid Fmoc-PfpGly-OH was synthesized to analog protocols accordingly.

### Synthesis of the CH₂F-CH₂-/CHF₂-alkylated Glycine-Ni(II) Ligand Complex 2 & 3

The chiral Ni ligand (20.00 g, 33.3 mmol, 1.00 equiv.) and 1-fluoro-2-iodoethane (8.68 g, 5.85 mL, 49.9 mmol, 1.50 equiv.) / 1,1-difluoro-2-iodoethane (9,57 g, 4.39 mL, 49.9 mmol, 1.50 equiv.) were dissolved in dried and freshly degassed dimethylformamide (200 mL) under inert conditions. The reaction mixture was cooled down to 0 °C. Potassium hydroxide (1.96 g, 34.9 mmol, 1.05 equiv.) was dissolved in dried and freshly degassed MeOH (18 mL) and added dropwise to the reaction mixture. The mixture was stirred for 3 h at 0 °C for the synthesis of the mono- and difluorinated compounds. Water (64 mL) was added and stirred for 1 h at room temperature. Subsequently, water (36 ml) was again added, and stirred for 1 h at room temperature. The reaction mixture was filtered and washed with a mixture of dimethylformamide/water (2:1, 36 mL) and water (40 mL). Finally, the purified compounds were dried *in vacuo* at 55 °C. The compounds **2** and **3** were obtained as red coloured solid (**2**, 16.24 g, 25.1 mmol, 75%) or purple coloured solid (**3**, 17.08 g, 25.7 mmol, 77%).

### CH₂F-CH₂-alkylated Glycine-Ni(II) Ligand Complex 2:

**¹H NMR (600 MHz, METHANOL-*D*₄)** δ = **8.81** (d, J = 2.1 Hz, 1H), **8.26** - **8.24** (m, 1H), **7.97** (d, J = 9.3 Hz, 1H), **7.66** - **7.62** (m, 1H), **7.59** (t, J = 7.6 Hz, 1H), **7.52** (t, J = 6.5 Hz, 1H), **7.47** (d, J = 8.3 Hz, 1H), **7.43** (d, J = 7.5 Hz, 1H), **7.10** (dd, J = 9.3, 2.6 Hz, 1H), **7.02** (dt, J = 7.7 Hz, 1H), **6.54** (d, J = 2.6 Hz, 1H), **4.91 - 4.82** (m, 1H), **4.81 - 4.71** (m, 1H) **4.18** (d, J = 12.6 Hz, 1H), **4.04** (dd, J = 3.7 Hz, 1H), **3.58** - **3.50** (m, 2H), **3.39** - **3.34** (m, 2H), **2.66** - **2.57** (m, 2H), **2.24 - 2.13** (m, 2H), 2.10 - 2.01 (m, 1H) ppm.

**¹³C {1H} NMR (151 MHz, METHANOL-D4):** δ = 181.29, 180.13, 171.28, 140.61, 136.61, 133.31, 133.00, 132.73, 131.89, 131.73, 131.10, 130.80, 130.23, 129.24, 129.14, 127.79, 127.62, 127.02, 125.53, 124.22, 80.20, 79.10, 71.36, 67.49, 62.57, 58.27, 34.88, 30.59, 23.03 ppm.

**¹⁹F NMR (565 MHz, METHANOL-D4): δ** = -218.30 - -218.58 (m, 3F) ppm.

### CHF₂-CH₂-alkylated Glycine-Ni(II) Ligand Complex 3:

**¹H NMR (600 MHz, METHANOL-D₄)** δ = **8.79** (d, *J* = 2.1 Hz, 1H), **8.27** (dd, *J* = 8.2 Hz, 1H), **7.98 -7.94** (m, 1H), **7.60-7.41** (m, 2H), **7.55 - 7.50** (m, 2H), **7.48 -7.44** (m, 2H), **7.11** (dd, *J* = 11.9 Hz, 1H), **7.04** (d, *J* = 1.5 Hz, 1H), **6.54** (d, *J =* 2.6 Hz, 1H), **4.18** (d, *J* = 12.5 Hz, 1H), **4.09** - **4.03** (m, 1H), **3.70** - **3.48** (dd, *J* = 13.7, 4.7 Hz, 2H), **3.41** - **3.33** (m, 2H), **2.66** - **2.56** (m, 2H), **2.42** (td, 2J = 9.6 Hz, 3J = 6.2 Hz, 1H), **2.26** - **2.08** (m, 3H) ppm.

**¹³C {1H} NMR (151 MHz, METHANOL-D4):** δ = 181.30, 179.35, 171.94, 140.76, 136.58, 133.29, 133.17, 132.86, 132.74, 131.95, 131.91, 131.12, 130.83, 130.40, 129.85, 129.37, 129.24, 128.42, 127.56, 126.97, 125.55, 124.27, 114.95, 71.28, 62.55, 58.31, 30.55, 30.50, 23.14.

**¹⁹F NMR (565 MHz, METHANOL-D4): δ =** -155.45 - -117.53 (m, 3F) ppm

### Synthesis of Fmoc-MfeGly-OH (4) & Fmoc-DfeGly-OH (5)

Compound **2/3** (15 g, 0.023 mol [**2**] / 0,022 mol [**3**], 1 equiv.) was dissolved in dimethoxyethane (110 mL) and 3N HCI (66.5 mL) and heated to 60 °C while stirring for 3h. Then the mixture was filtered and washed with water (30 mL). The combined solutions were concentrated, leading to further precipitation of Ni-ligand residues. These precipitates were filtered and washed with water (25 mL) and the solutions were combined. EDTA-(Na₂) (8.56 g, 0.023 mol, 1 equiv.) in MeCN (55 mL) was added and the mixture was stirred for 2 h at 23 °C. The reaction was quenched by adjusting the pH to 8-8.5 by use of a 48%-NaOH solution. Afterwards, Na₂CO₃ (4.9 g, 0.046 mol, 2 equiv.) was supplemented and Fmoc-OSu (7.75 g, 0.023 mol, 1 equiv.) in acetone (45 mL) was added slowly while the reacting mixture was stirred at 23 °C for 20 h. Then, MeCN and acetone were removed from the solution under reduced pressure. The aqueous solution was washed with Et₂O (3 * 30 mL) to remove leftovers of Fmoc-OSu. Then, the pH of the solution was adjusted to 2 by using HCl_{conc}. before the aqueous phase was extracted with ethyl acetate (6 * 50 mL). The combined organic phases were dried over Na2SO4, filtered, concentrated, and dried *in vacuo* at 55 °C. The crude product (about 8.5 g) was dissolved in ethyl acetate (30 mL) and toluene (150 mL) and warmed to 75 °C for complete dissolution. Then, the solution was concentrated under reduced pressure and after further addition of toluene (75 mL) concentrated again to a total volume of approximately 100 mL. This solution was left to stand overnight at room temperature, leading to precipitation of pure Fmoc-protected amino acids MfeGly **(4)** and DfeGly **(5).** The amino acid was filtered, washed with ice-cold toluene (100 mL) and hexane (100 mL). Fmoc-MfeGly-OH **(4)** was washed additionally with ice-cold DCM (20 mL) to remove impurities. Both products were afterwards dried *in vacuo* at 55 °C. Compounds **4** and **5** were both obtained as white solid substances (4.34 g, 0.0126 mol, 55% **[4]** and 6.59 g, 0.0182 mol, 83% **[5]**)**.**

### Fmoc-MfeGly-OH (4):

**¹H NMR (600 MHz, METHANOL-D₄):** δ = **7.76** (d, J = 7.6 Hz, 2H), 7.64 (t, J = 7.5 Hz, 2H), 7.35 (t, J = 7.5 Hz, 2H), 7.27 (t, J = 7.4 Hz, 2H), **4.56** - **4.40** (m, 2H), **4.37** - **4.31** (m, 2H), **4.31 - 4.25** (m, 1H), 4.19 (t, J = 7.1 Hz, 1H), **2.31 - 2.21** (m, 1H), **2.03 -1.92** (m, 1H).

**¹³C {1H} NMR (151 MHz, METHANOL-D4):** δ = 173.98, 157.36, 143.99, 143.85, 141.27, 127.45, 126.84, 126.81, 124.93, 119.58, 80.59, 79.50, 66.66, 50.51, 32.18, 32.05.

**¹⁹F NMR (565 MHz, METHANOL-D4):** δ = -222.46 - -222.83 (m, 3F)

### Fmoc-DfeGly-OH (5):

**¹H NMR (600 MHz, METHANOL-D₄):** δ = **7.77** (d, J = 7.6 Hz, 2H), **7.67** - **7.62** (m, 2H), **7.36** (t, J = 7.0 Hz, 2H), **7.28** (td, J = 7.4, 1.2 Hz, 2H), **6.03** - **5.81** (m, 1H), **4.41** - **4.31** (m, 2H), **4.35** - **4.27** (m, 1H), **4.20** (t, J = 6.9 Hz, 1H), **2.43** - **2.33** (m, 1H), **2.26** - **2.16** (m, 1H).

**¹³C {1H} NMR (151 MHz, METHANOL-D4):** δ = 172.75, 157.13, 143.95, 143.83, 141.28, 127.46, 126.82, 124.92, 119.58, 117.30, 115.72, 66.70, 35.84, 35.69, 35.54, 24.93.

**¹⁹F NMR (565 MHz, METHANOL-D4):** δ = -117.45 - -119.06 (m, 3F).

### Synthesis of Ac-[2]Abz-Gly-OH (6)

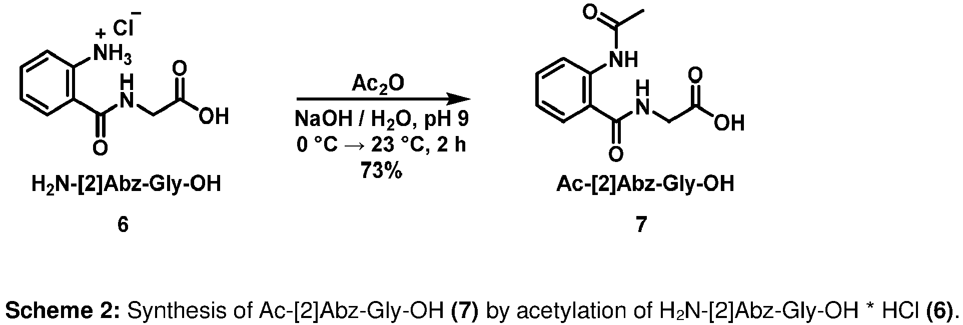

As shown in **Scheme S2,** The synthesis of Ac-[2]Abz-Gly-OH **(8)** comprises a single-step synthetic strategy by treating the commercially available H₂N-[2]Abz-Gly-OH * HCl **(7)** with a standard acetylation-protocol.

H2N-[4]Abz-Gly-OH * HCl (250 mg, 1.083 mmol, 1 equiv.) was dissolved in MilliQ-H₂O (3 mL). Then, the pH was adjusted to 9 with a 1M NaOH solution. The mixture was cooled to 0 °C and freshly distilled acetic anhydride (0.20 mL, 5.14 mmol, 2 equiv.) was added slowly. The pH was again adjusted to 9 and the reacting mixture was stirred for 2 h while warming to 23 °C. Thereupon HCl_{conc} Was added slowly until a pH of 2 was reached. The reaction mixture was extracted with ethyl acetate (5 × 5 mL). The organic layers were combined, dried over Na₂SO₄, filtered, concentrated, and dried *in vacuo*.Compound **8** was obtained as a yellow powder (186.7 mg, 0.79 mol, 73%).

**¹H NMR (600 MHz, METHANOL-D₄):** δ = **8.92** (d, *J* = 9.4 Hz, 1H), **8.29** (dd, *J* = 7.9, 1.5 Hz, 1H), **8.09** (d, J = 9.0 Hz, 1H), **7.81 - 7.77** (m, 1H), **4.70** (s, 2H), **2.77** (s, 3H).

**¹³C {1H} NMR (151 MHz, METHANOL-D4):** δ = 172.50, 170.87, 170.50, 138.56, 132.30, 128.24, 124.00, 123.18, 122.06, 41.47, 23.92.

### Characterization of Fmoc-PfpGly-OH (8)

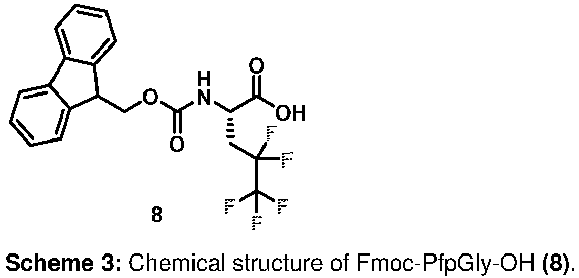

**¹H NMR (600 MHz, METHANOL-D₄):** δ = **8.41** (d, J = 7.6 Hz, 2H), **8.28** (d, J = 7.6 Hz, 2H), **8.00** (t, J = 7.5 Hz, 2H), **7.92** (t, J = 7.5 Hz, 2H), **5.18** (d, J = 9.9 Hz, 1H), **4.97** (d, J = 7.1 Hz, 1H), **4.84** (d, J = 7.1 Hz, 1H), **3.48** - **3.37** (m, 1H), **3.32** - **3.21** (m, 1H).

**¹³C {1H} NMR (151 MHz, METHANOL-D4):** δ = 172.60, 157.47, 144.45, 141.86, 128.14, 127.44, 125.62, 120.12, 67.57, 32.13, 25.75.

**¹⁹F NMR (565 MHz, METHANOL-D4):** δ = -86.67 (s), -118.21 (ddd, J = 267.3, 26.1, 8.5 Hz).

This amino acid was synthesized according to recently published protocols by Koksch and coworkers (T. Hohmann, M. Dyrks, S. Chowdhary, M. Weber, D. Nguyen, J. Moschner and B. Koksch, The Journal of Organic Chemistry, 2022, 87, 10592-10604.)

### Synthesis and purification of peptides

All peptides were synthesized with a microwave-equipped Liberty Blue^{™} peptide synthesizer (CEM, Matthews, NC, USA). A Rink Amide ProTide^{™} resin (CEM, Matthews, NC, USA) was utilized and the synthesis was performed either in 0.05 mmol or 0.1 mmol scale using Oxyma/DIC as activating reagents. Detailed coupling protocols are listed in the supporting information. Acetylation was done manually in three batches using acetic anhydride (10% v/v) and DIPEA (10% v/v) in DMF (6 mL).

All peptides were cleaved from the resin by treatment with TFA /TIPS/H₂O (90/5/5) [1 ml cleavage-cocktail per 50 mg resin] for three hours using sonication at room temperature. Then the resins were washed with TFA and DCM, and excess of solvents were removed by evaporation. Peptides were dried by lyophilization before purification with preparative reversed phase HPLC. Purification of synthesized peptides was performed on a Knauer low-pressure HPLC system (Knauer GmbH, Berlin, Germany) sold by VWR (Darmstadt, Germany), comprising a LaPrep Sigma preparative pump (LP1200), a ternary low-pressure gradient, a dynamic mixing chamber, a 6-port-3-channel injection valve with an automated preperative 10 mL sample loop, a LaPrep Sigma standard 1-channel-UV-detector (LP3101), a flow cell with 0.5mm thickness and a 16-port LaPrep Sigma fractionation valve (LP2016). A Kinetex RPC18 endcapped (5 µM, 100 Å, 250 × 21.2 mm, Phenomenex^{®}, USA) HPLC-column was used. A Security GuardTM PREP Cartridge Holder Kit (21.20 mm, ID, Phenomenex^{®}, USA) served as pre-column. As eluents water and ACN, both containing 0.1% (v/v) TFA were applied. HPLC runs were performed with a flow rate of 15.0 mL/min, UV-detection occurred at 220 nm for respective peptides. Data analysis occurred with an EZChrom Elite-Software (Version 3.3.2 SP2, Agilent). After separation, the purity of the collected fractions was determined by analytical HPLC. Analytical HPLC was carried out on a Chromaster 600 bar DAD-System with CSM software or a Hitachi Primaide^{™} UV-HPLC system (both from VWR/Hitachi, Darmstadt, Germany). A Kinetex^{®} RP-C18 (5 µM, 100 Å, 250 × 4.6 mm, Phenomenex^{®}, USA) column and a SecurityGuard^{™} Cartridge Kit equipped with a C18 cartridge (4 × 3.0mm, Phenomenex^{®}, USA)) as pre-column was used. Otherwise, a Luna^{®} RP-C8 (5 µm, 100 Å, 150 × 3 mm, Phenomenex^{®}, USA) column was used. As eluents water and ACN, both containing 0.1% (v/v) TFA were applied. A flow rate of 1 mL/min was used and UV-detection occurred at 220 nm or 280 nm for respective peptides. Data analysis was done with EZ Chrom ELITE software (version 3.3.2, Agilent). The resulting pure peptides (>95%) were obtained after lyophilization of the collected fractions.

### Peptide AbuR14

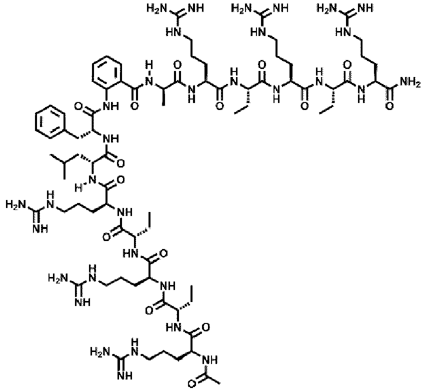

The purity of this peptide after synthesis and isolation was determined through analytical HPLC with DAD detection at 220 nm. HPLC: Hitachi Primaide with Kinetex^{®} C18 RP-column / solvent: **(A)** H2O + 0.1% TFA / **(B)** ACN + 0.1% TFA / gradient: 10% (B) **→** 70% (B) over 18 min.

### Peptide MfeGlyR14

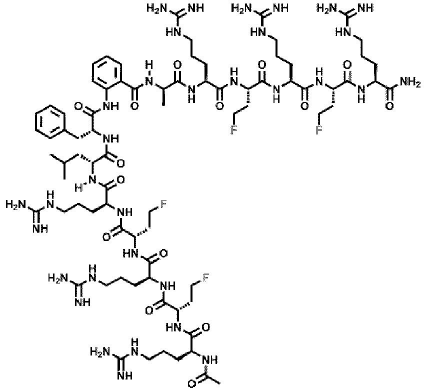

The purity of this peptide after synthesis and isolation was determined through analytical HPLC with DAD detection at 220 nm. HPLC: Hitachi Primaide with Kinetex^{®} C18 RP-column / solvent: **(A)** H2O + 0.1% TFA / **(B)** ACN + 0.1% TFA / gradient: 10% (B) **→** 70% (B) over 18 min.

### Peptide DfeGlyR14

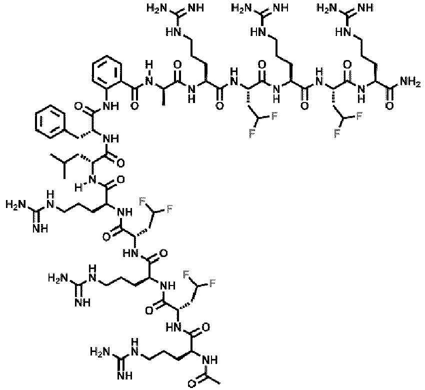

The purity of this peptide after synthesis and isolation was determined through analytical HPLC with DAD detection at 220 nm. HPLC: Hitachi Primaide with Kinetex^{®} C18 RP-column / solvent: **(A)** H2O + 0.1% TFA / **(B)** ACN + 0.1% TFA / gradient: 10% (B) **→** 70% (B) over 18 min.

### Peptide TfeGlyR14

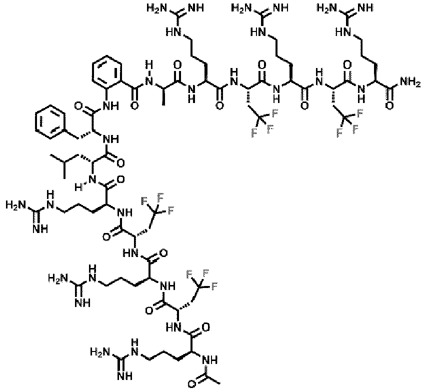

The purity of this peptide after synthesis and isolation was determined through analytical HPLC with DAD detection at 220 nm. HPLC: Hitachi Primaide with Kinetex^{®} C18 RP-column / solvent: **(A)** H2O + 0.1% TFA / **(B)** ACN + 0.1% TFA / gradient: 10% (B) **→** 70% (B) over 18 min.

### Peptide SAJO-2

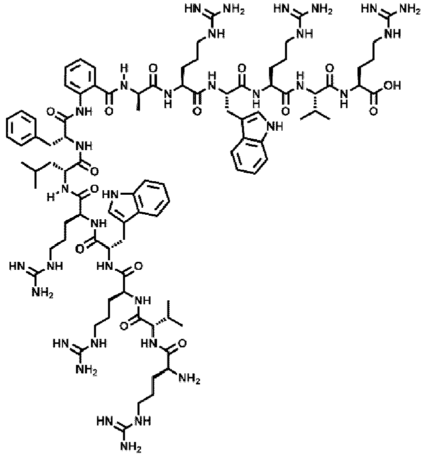

The purity of this peptide after synthesis and isolation was determined through analytical HPLC with DAD detection at 280 nm. HPLC: Hitachi Primaide with Kinetex^{®} C18 RP-column / solvent: **(A)** H2O + 0.1% TFA / **(B)** ACN + 0.1% TFA / gradient: 10% (B) **→** 70% (B) over 18 min.

### Peptide SAJO-Abu

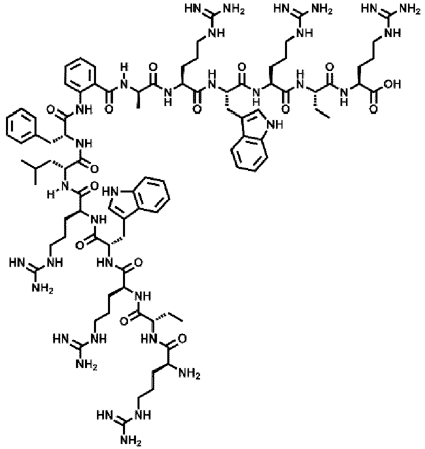

The purity of this peptide after synthesis and isolation was determined through analytical HPLC with DAD detection at 220 nm. HPLC: Hitachi Primaide with Kinetex^{®} C18 RP-column / solvent: **(A)** H2O + 0.1% TFA / **(B)** ACN + 0.1% TFA / gradient: 10% (B) **→** 60% (B) over 18 min.

### Peptide SAJO-TfeGly

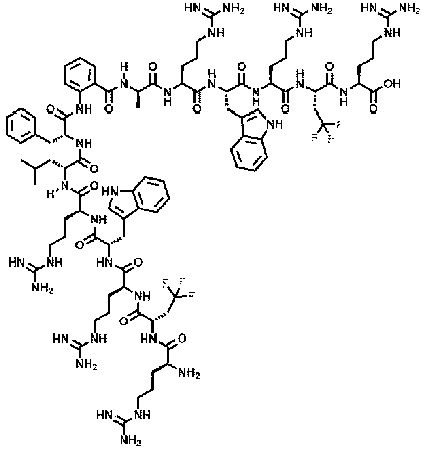

The purity of this peptide after synthesis and isolation was determined through analytical HPLC with DAD detection at 220 nm. HPLC: Hitachi Primaide with Kinetex^{®} C18 RP-column / solvent: **(A)** H2O + 0.1% TFA / **(B)** ACN + 0.1% TFA / gradient: 10% (B) **→** 60% (B) over 18 min.

### Peptide SAJO-PfpGly

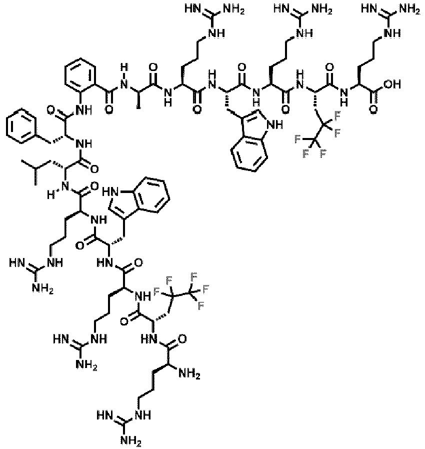

The purity of this peptide after synthesis and isolation was determined through analytical HPLC with DAD detection at 220 nm. HPLC: Hitachi Primaide with Kinetex^{®} C18 RP-column / solvent: **(A)** H2O + 0.1% TFA / **(B)** ACN + 0.1% TFA / gradient: 10% (B) **→** 60% (B) over 18 min.

### Antimicrobial susceptibility testing

Minimum inhibitory concentrations (MIC) assays were performed according to Clinical and Laboratory Standards Institute (CLSI) recommendations in cation-adjusted Mueller-Hinton broth (Mueller-Hinton II) broth in flat-bottomed, 96-well plates (Corning, Wiesbaden, Germany) with an inoculum of 10⁵ bacteria/well and incubated at 37°C overnight. The optical density of the cultures was determined at 460 and 600 nm immediately after inoculation and after overnight incubation using a BioTek Synergy HT plate reader. Controls included bacterial strains, medium alone and wells without peptide additions. A serial dilution of each peptide was done before bacteria addition. A serial dilution of media including the respective peptide solvent without peptides supplement (Dimethylsulfoxid; Sigma-Aldrich, Taufkirchen, Germany or 1 x phosphate buffered saline; Biochrom, Berlin, Germany) functions as control to ensure the solvent influence on growth.

Table 1 shows antibacterial activities of peptides AbuR14, MfeGlyR14, DfeGlyR14 and TfeGlyR14.

**Table 1: Antibacterial activities of peptides AbuR14, MfeGlyR14, and TfeGlyR14**

| **Peptide** | **1*MIC [µg/mL]** | | | | | | |
|---|---|---|---|---|---|---|---|
| | ***S*. Typhimurium** | ***E. coli*** | ***E*. *faecalis*** | ***S*. *aureus*** | ***P*. *aeruginosa*** | ***K. pneumoniae*** | ***C*. *albicans*** |
| | **ATCC 14028** | **ATCC 25922** | **ATCC 29212** | **ATCC 29213** | **ATCC 27853** | **ATCC 700603** | **IMT 9655** |
| **AbuR14** | 256 | 128 | 512 | ≥1024 | ≥1024 | ≥1024 | 512 |
| **MfeGlyR14** | 1024 | 512 | 1024 | ≥1024 | ≥1024 | ≥1024 | 1024 |
| **DfeGlyR14** | 128 | 256 | 1024 | 512 | ≥1024 | 1024 | 512 |
| **TfeGlyR14** | 128 | 64 | 128 | 128 | 1024 | 512 | 128 |

Peptide-induced membrane disrupting activity was determined before by calculation of EC₅₀ values and an increase in bioactivity with a growing degree of side chain fluorination was found. **AbuR14** possesses the least membrane disrupting properties (EC₅₀ = 148 µM). Substitution of **Abu** with **MfeGly** reduces the EC₅₀ to about 41% in comparison to its native counterpart, indicating a significant increase in membrane activity upon fluorination without affecting peptide hydrophobicity. A growing content in side chain fluorination leads for **DfeGlyR14** (EC₅₀ = 77 µM) to a comparably minor change in EC₅₀ than for **MfeGlyR14** (EC₅₀ = 88 µM), but for **TfeGlyR14** (EC₅₀ = 47 µM) to a minimum 3-fold optimization of membrane activity than **AbuR14.** The AMP with the strongest hydrophobicity among the **XR14**-series was found as the most potent peptide to penetrate the anionic lipid bilayers and was surprisingly found to possess superior properties than the reference **SAJO-2** (EC₅₀ = 70 µM) in this context.

Hemolysis assays were performed on red blood cells and cytotoxicity tests with two cell lines (HeLa, A549). No potent hemolytic activity for all peptides up to a concentration of 250 µg/mL, corresponding to concentrations of 140 µM **(AbuR14)** - 125 µM **(TfeGlyR14)** was determined (data not shown). Further measurements revealed peptide-induced hemolysis for the most fluorinated variant **TfeGlyR14** at minimum concentrations of 1 mg/mL (0.5 mg/mL for **SAJO-**2), which is in accordance to prior observed trends of fluorine-containing antibiotic peptides *(data not shown).* Furthermore, all peptides showed exhibited zero cytotoxicity up to 250 µg/mL concentration towards both cell lines. These results indicated that the overall content of fluorine accompanied by enhanced hydrophobicity throughout the cationic peptide sequence was pivotal in regulating membrane permeabilization of artificial phospholipid membranes at physiological pH without affecting blood or human cells.

Experimentally determined MIC data (Table 1) revealed an increase in antimicrobial activity upon CH₃ to CF₃ substitution on the hydrophobic amino acid side chain. Herein, partially fluorinated residues (MfeGly, DfeGly) were found to possess less or equal potent activities. A loss in hydrophobicity due to enhanced fluorine-induced polarity is considered to govern the beneficial effects of this element on the peptide's bioactivity (S. Chowdhary, R. F. Schmidt, A. K. Sahoo, T. tom Dieck, T. Hohmann, B. Schade, K. Brademann-Jock, A. F. Thünemann, R. R. Netz, M. Gradzielski, B. Koksch, Nanoscale 2022, 14, 10176-10189).

Table 2 shows antibacterial activities of peptides SAJO-2, SAJO-Abu, SAJO-TfeGly und SAJO-PfpGly.

**Table 2: Antibacterial activities of peptides SAJO-2, SAJO-Abu, SAJO-TfeGly und SAJO-PfpGly.**

| **Peptide** | **1*MIC [µg/mL]** | | | | | | |
|---|---|---|---|---|---|---|---|
| | ***S*. Typhimurium** | ***E. coli*** | ***E*. *faecalis*** | ***S*. *aureus*** | ***P*. *aeruginosa*** | ***K. pneumoniae*** | ***C***. ***albicans*** |
| | **ATCC 14028** | **ATCC 25922** | **ATCC 29212** | **ATCC 29213** | **ATCC 27853** | **ATCC 700603** | **IMT 9655** |
| **SAJO-2** | 16 | 8 | 64 | 64 | 64 | 32 | 32 |
| **SAJO-Abu** | 32 | 16 | 256 | 64 | 128 | 64 | 32 |
| **SAJO-TfeGly** | 32 | 8 | 64 | 64 | 64 | 64 | 32 |
| **SAJO-PfpGly** | 16 | 4 | 32 | 16 | 64 | 16 | 32 |

The reference **SAJO-2** is reported as a bioactive peptide with a broad spectrum of antibacterial activity and low rates of hemolysis. (H. Khandelia and Y. N. Kaznessis, The Journal of Physical Chemistry B, 2007, 111, 242-250).

It was anticipated that the substitution of both valine residues either with **TfeGly** or, ultimately, **PfpGly,** to induce fluorine-based beneficial effects on cell-damaging interactions between the **SAJO**-motif with bacterial membranes.

MIC data of the **SAJO**-peptides (see **table 2)** revealed an overall superiority of this structural pattern in comparison to the **XR14**-series. These different trends underline the ability of Trp residues to conduct the peptides action mechanism with regards to its strong membrane-disruptive activity.

Almost similar MIC values found for **SAJO-2** and **SAJO-TfeGly** do harmonize with their analogous non-polar character, emphasizing hydrophobic interactions as main driving force in AMP activity regardless about the degree of side chain fluorination. Moreover, partially inferior activities of the fluorinated variant **SAJO-TfeGly** than its native counterpart **SAJO-2** seem to dispute the presence of fluorine-specific beneficial effects. Following this analogy, the less hydrophobic peptide **SAJO-Abu** revealed similar or (up to 4-fold) reduced potencies than its (-CF₃)-substituted derivative.

With regards to antifungal activity, the substitution of the hydrophobic residues led to retained activities towards *C. albicans,* very different from the observation for the **XR14**-series. An interesting result was constituted by **SAJO-PfpGly** which showed the highest activity towards gram-positive microbes (*S*. *aureus)* among all peptides studied in this work, but also analog MIC values than **SAJO-2** against some gram-negative species (P. *aeruginosa).* The most improved activity was found, consequently, for the derivative whose structure contained the most hydrophobic aliphatic side chain. Taking the analogous data set obtained for **SAJO-2** and **SAJO-TfeGly** into account, it can be concluded that the utilization of fluorinated side chains for this peptide motif as an approach to improve antimicrobial activity rather as a function of enhanced hydrophobicity than fluorine-specific interactions

### Peptide Sajo-PfpGly was tested further against the following E.coli strains:

| ***E. coli* strain** | **MIC [µg/mL]** - **SAJO-PfpGly** | **notes** |
|---|---|---|
| IMT 51466 (E. coli clinicial isolate, horse) | 4 | ESBL |
| IMT 51684 | 8 | Mucoid, not MDR! |
| IMT 51773 (E. coli clinicial isolate, dog) | 8 | MDR |
| IMT 47271 | 16 | ESBL |
| IMT 47528 | 8 | MDR, mecA pos. |
| IMT 49981 | 16 | MDR, carbapenemase pos. |
| IMT 50717 | 16 | MDR |
| IMT 47260 | 8 | ESBL |
| IMT 50611 | 16 | Very mucoid, not MDR! |
| IMT 51705 (E. coli clinical isolate, dog) | 8 | MDR |
| IMT 51747 (E. coli clinical isolate, dog) | 16 | MDR |
| IMT 47192 | 16 | ESBL, NDM |
| IMT 47210 | 16 | MDR, CTX pos. |
| IMT 47690 | 16 | MDR |
| IMT 48045 | 8 | MDR, CTX pos. |
| IMT 51899 (E. coli clinicial isolate, rabbit) | 16 | Mucoid, ESBL₊ very res. |
| IMT 49099 | 16 | ESBL |
| IMT 50831 | 16 | MDR |
| IMT 51214 (E. coli clinical isolate, human,) | 32* | ESBL |
| IMT 49043 | 16 | MDR, carbapenemase pos., blaOXA-48 OXA-48 pos. |
| IMT 49908 | 16 | ESBL |
| IMT 50918 | 16 | MDR |
| | | |

| | | |
|---|---|---|
| **growth of 16 µg*/*mL not consistent for all samples* *ESBL: extended spectrum β-lactamase* *MDR: multi drug resistant* *mecA: resitance gene for penicillin-like antibiotics* *carbapenemase: group of β-lactamase* *NDM: New Dheli metallo-β-lactomose* *CTX: CTX-M-type β-lactamase* *blaOXA-48 OXA-48: carbapenemase genes* | | |

### Proteolytic digestion assay

For real-time monitoring of enzymatic digestion, peptides **AbuR14, TfeGlyR14, SAJO-2, SAJO-TfeGly** and **SAJO-PfpGly** were dissolved in 10 mM phosphate buffer, pH 7.4 (125 µL) at a two-fold concentration of 200 µM and gently mixed to obtain a homogeneous mixture. Afterwards, 125 µL of β-trypsin (0.015 µM, dissolved in 10 mM phosphate buffer, pH 7.4) were added and the samples were gently mixed for 5 seconds. The final reaction mixture comprised 100 µM peptide and 0.0075 µM enzyme, respectively. All samples were incubated at 30 °C over a period of 3 h. Aliquots of 40 µL were taken at fixed time points and quenched with 40 µl of a solution of 5% TFA in a water-methanol mixture [1:1] containing 100 µM Ac-[2]Abz-Gly-OH as reference. Afterwards, peptide degradation was monitored by HPLC analysis and remaining amounts of peptide were calculated in accordance with the reference signal. All experiments were performed in triplicates.

For the detection of peptide fragments derived from peptide proteolysis, all peptide samples were dissolved in 10 mM phosphate buffer, pH 7.4 (170 µL, 1 mM peptide concentration) and then were gently mixed to obtain a homogeneous solution. Afterwards, 35 µL of either α-chymotrypsin or β-trypsin (both 20 µM concentration stocks in 10 mM phosphate buffer, pH 7.4) were added and the samples were again gently mixed for 5 seconds. All samples were incubated at 30 °C over a period of 6 h. Aliquots of 25 µL were taken at fixed time points and quenched with 50 µL of a solution of 1% TFA in water containing 75 µM Ac-[2]Abz-Gly-OH as reference & 50 µL MeOH. Afterwards, peptide degradation was monitored by HPLC analysis. To identify the digestion profile of each peptide, each fragment cleaved from the full-length peptide was identified by ESI-ToF mass analysis on an Agilent 6220 ESI-ToF-MS spectrometer (Agilent Technologies, Santa Clara, CA, USA). All experiments were performed in triplicates

## Claims

1. Peptide with antimicrobial activity comprising the following sequence
Y¹-[R-X¹-R-X²-R-dL-dF-[2]Abz-dA-R-X²-R-X¹-R]-Y²
Wherein
- R is D-or L- Arginine or an Arginine analogue, dL is D-Leucine, dF is D-Phenylalanine, [2]Abz is 2-Aminobenzoic acid, dA is D-Alanine,
- X¹ is FₐH_{b}C - (F_{c}H_{d}C)ₙ -CH₂-CHNH₂CO₂H, wherein
a is 0,1, 2, 3; b is 0, 1, 2, 3; wherein the sum of a and b is always 3;
c is 0, 1, 2; d is 0, 1, 2; wherein the sum of c and d is always 2;
Wherein n is 0-5,
- X² is L-Tryptophan (W), D-Tryptophan or a Tryptophan analogue, or X¹,
- Y¹ is Acetyl or -H or any suitable protective group; and
- Y² is -NH₂ or -OH or any suitable protective group.

2. Peptide according to claim 1, **characterized in that**
a is 2 or 3; b is 0 or 1; wherein the sum of a and b is always 3;
c is 1 or 2; d is 0 or 1, wherein the sum of c and d is always 2.
Wherein n is 0, 1, 2, preferably n is 0 or 1; and
X² is L-Tryptophan (W), D-Tryptophan or a Tryptophan analogue or X¹.

3. Peptide according to one of the preceding claims, **characterized in that**
a is 3; b is 0; c is 2; d is 0,
Wherein n n is 0 or 1; and
X² is L-Tryptophan (W), D-Tryptophan or a Tryptophan analogue or X¹.

4. Peptide according to one of the preceding claims, **characterized in that**
a is 3; b is 0; c is 2; d is 0, Wherein n is 0 or 1; and
X² is L-Tryptophan (W),
Y¹ is H; and Y² is OH.

5. Peptide according to one of the preceding claims, **characterized in that** R is L-Arginine.

6. Peptide according to one of the preceding claims for use as an antimicrobial substance, in particular as an antibiotic or antifungal.

7. Peptide according to one of the preceding claims for use in a method of treatment of bacterial infections by gram-negative or gram-positive bacterial strains.

8. Peptide according to claim 7, **characterized in that** the bacterial infection is an infection by one of the genus of Staphylococcus, Escherichia, Pseudomonas, Klebisella.

9. Peptide according to one of claims 7-8, **characterized in that** the bacterial infection is an infection by one of *Staphylococcus aureus, Staphylococcus aureus, Escherichia coli, Escherichia faecalis, Klebisella pneumonia, Pseudomonas aeruginosa.*

10. Method for manufacturing a peptide according to one of the preceding claims comprising the following steps:
d) synthesizing a peptide by solid phase peptide synthesis on a solid phase peptide synthesis resin, the peptide comprising the structure as defined in claim 1,
e) cleaving the synthesized peptide from the solid phase peptide synthesis resin;
f) optionally purifying the synthesized and cleaved peptide.
